# EUROPÄISCHE PATENTSCHRIFT

(11) **EP 2 552 516 B1**
(45) Veröffentlichungstag und Bekanntmachung des Hinweises auf die Patenterteilung: **18.01.2017**
(21) Anmeldenummer: 11710663.3
(22) Anmeldetag: 22.03.2011
(51) Int. Cl.: A61M 5/19, B05C 17/005, B65D 81/32, F16L 19/00, A61M 39/10

(54) **LUER-KONNEKTOR MIT ÜBERWURFSCHRAUBE ZUM ANSCHLUSS AN EINE VERABREICHUNGSVORRICHTUNG**
LUER-CONNECTOR WITH UNION SCREW FOR CONNECTION TO A FLUID DELIVERY DEVICE
RACCORD LUER DOTÉ D'UNE VIS DE FIXATION, POUR RACCORDEMENT À UN DISPOSITIF D'ADMINISTRATION

(30) Priorität: 26.03.2010 CH 4462010
(43) Veröffentlichungstag der Anmeldung: 06.02.2013
(73) Patentinhaber: Medmix Systems AG, 6343 Rotkreuz (CH)
(72) Erfinder: KAYSER, Ralph Egon, CH-6004 Luzern (CH)
(74) Vertreter: Rutz, Andrea
(86) Internationale Anmeldenummer: PCT/CH2011/000057
(87) Internationale Veröffentlichungsnummer: WO 2011/116484

(56) Entgegenhaltungen:
- EP-A1- 0 775 501
- EP-A1- 1 552 858
- EP-A2- 0 953 365
- WO-A1-2010/009563
- WO-A1-2010/020061
- WO-A1-2010/061234
- DE-U1-202004 012 714
- US-A1- 2007 129 705

## Beschreibung

### TECHNISCHES GEBIET

Die vorliegende Erfindung betrifft ein Anschlussteil zum Anschluss an eine Austrageinheit. Das Anschlussteil weist zumindest ein Kupplungsteil auf, mittels welchem das Anschlussteil an eine Austrageinheit anschliessbar ist. Das Kupplungsteil umfasst einen konischen Verbindungsbereich sowie eine Verriegelungsstruktur.

### STAND DER TECHNIK

Austrageinheiten wie beispielsweise Spritzen zum Verabreichen von Medikamenten an Patienten sind seit langem allgemein bekannt und werden in der Medizin oft benutzt. Eine handelsübliche Spritze weist ein Spritzengehäuse mit einer distalen Austrittsöffnung und einen Kolben auf, der von einem proximalen Ende her in ein Reservoir des Spritzengehäuses einschiebbar ist. Das Reservoir dient dabei zur Aufnahme eines fluiden Produktes, zum Beispiel eines Medikamentes. An die Austrittsöffnung ist ein Anschlussteil wie beispielsweise eine Injektionskanüle oder ein Verbindungsschlauch anschliessbar. Im Bereich der Austrittsöffnung ist dazu in der Regel ein Kupplungsteil ausgebildet, welches zum Anschluss von verschiedenartigen Anschlussteilen dient. Zur Verbindung von Anschlussteilen und Spritzen hat sich in der Medizin die sogenannte Luer-Verbindung durchgesetzt. Dabei weist eines der zu verbindenden Teile, üblicherweise die Spritze, einen männlichen Konusbereich und das andere Teil einen dazu komplementär ausgebildeten, weiblichen Konusbereich auf. Der weibliche Konusbereich des Anschlussteils ist dann auf den männlichen Konusbereich der Spritze aufschiebbar, wodurch eine fluiddichte Verbindung zwischen dem Reservoir der Spritze und dem Innern des Anschlussteiles entsteht. Zur Sicherung des Anschlussteiles an der Spritze weist das Kupplungsteil zudem oft eine Sicherungshülse mit einem Innengewinde auf, in welches ein am Anschlussteil um den weiblichen Konusbereich herum angebrachtes Aussengewinde einschraubbar ist.

Die Konusse von derartigen Luer-Verschlüssen weisen üblicherweise eine Steigung von 6 % auf. Luer-Verschlüsse sind zudem in den Normen DIN EN 1707:1996 und DIN EN 20594-1:1993 im Detail beschrieben und entsprechend standardisiert.

Wenn die Sicherungshülse starr mit dem Spritzengehäuse verbunden ist, bedingt das eine gegenseitige Drehung der beiden zu verbindenden Konusbereiche. Dies ist beispielsweise im Fall einer Doppelkolbenspritze problematisch, bei der das Spritzengehäuse zwei separate Reservoirs mit jeweils einer Austrittsöffnung und einem Kupplungsteil aufweist. Ein Anschlussteil mit zwei dazu komplementär ausgebildeten Kupplungsteilen ist dann nicht an die Doppelkolbenspritze anschliessbar, da die beiden Konusbereiche der Doppelkolbenspritze starr miteinander verbunden sind. Ein einfacheres Sichern der Luer-Verbindung ist ermöglicht, wenn die Sicherungshülse drehbar am männlichen Konus angebracht ist, da dann die beiden zu verbindenden Teile beim Zusammenschrauben der beiden Gewinde nicht gegeneinander verdreht werden müssen. Die Dokumente US 4,629,455 und US 5,702,374 beschreiben derartige Vorrichtungen, bei denen die Sicherungshülse jeweils drehbar am männlichen Konus angebracht ist.

Im Dokument WO 2010/009563 ist ein Anschlusselement gezeigt, welches an zwei Spritzen anschliessbar ist, die durch eine Haltevorrichtung zusammengehalten sind und dadurch gemeinsam eine Doppelkolbenspritze bilden.

Die WO 2010/020061 beschreibt eine Austragvorrichtung, welche zwei Reservoirs mit jeweils einem Auslass aufweist. An diese beiden Auslässe ist mittels einer Rastverbindung ein Anschlussstück anschliessbar, in welchem beim Austragen die in den Reservoirs aufgenommenen Substanzen miteinander vermischt werden.

Das Dokument DE 37 37 665 beschreibt einen Luer-Verschluss, bei dem das gesamte Kupplungsteil mit dem männlichen Luer-Konus und der Sicherungshülse drehbar an einem zu verbindenden Rohr angebracht sind. Ein gegenseitiges Verdrehen des männlichen und des weiblichen Konusbereiches ist jedoch deshalb nachteilig, weil dabei eine Reibungskraft zwischen den beiden konischen Bereichen auftritt, welche das Zusammenschrauben der beiden Kupplungsteile für den Benutzer erschwert. Ein sattes Zusammenschrauben der beiden Gewinde ist jedoch erwünscht, weil sich dabei die beiden zu verbindenden Kupplungsteile verstärkt aufeinander pressen und sich dadurch die Dichtwirkung der Verbindung verbessert.

Weitere Luer-Verschlüsse sind in den Dokumenten US 5,984,373, WO 2009/093249, US 2006/0157984 und DE 86 10 008 beschrieben.

Oft ist es jedoch wünschenswert, Anschlussteile herzustellen, welche auf eine möglichst einfache Art und Weise an bereits bestehende Spritzen oder andere Austrageinheiten, wie zum Beispiel Doppel- oder Mehrfachkolbenspritzen oder auch Kartuschen, anschliessbar sind. Da bei vielen dieser Austrageinheiten die Sicherungshülse jedoch nicht drehbar ist, ist es weiterhin notwendig, dass das Anschlussteil als Ganzes gegenüber der Austrageinheit beim Zusammenschrauben der beiden Gewinde verdreht werden muss, wenn das Kupplungsteil des Anschlussteiles gemäss dem Stand der Technik ausgebildet ist. Je nach Funktion und Ausgestaltung des Anschlussteiles kann dies einen erheblichen Nachteil darstellen oder sogar den Anschluss an eine derartige Austrageinheit verunmöglichen.

In den Dokumenten US 2007/0129705, EP 0 775 501, EP 0 953 365 und EP 1 552 858 sind Anschlusselemente offenbart, bei welchen jeweils ein Verriegelungselement um einen Luer-Konus herum angebracht ist. Das Verriegelungselement ist dabei jeweils drehbar zum Luer-Konus angeordnet und weist eine Verriegelungsstruktur auf, um das Anschlusselement und ein daran angeschlossenes Gegenstück gegenseitig zu verriegeln.

In der DE 20 2004 012 714 ist ein Luer-Lock-Verbinder offenbart, bei welchem am männlichen Konusbereich eine drehbare Überwurfmutter angebracht ist, um eine Schraubverriegelung zwischen dem männlichen und dem weiblichen Anschlussteil zu ermöglichen.

Die WO 2010/061234 zeigt ein Anschlusselement, bei welchem eine Drehhülse mit einem Aussengewinde um einen weiblichen Luerkonus herum angebracht ist.

### DARSTELLUNG DER ERFINDUNG

Es ist eine Aufgabe der vorliegenden Erfindung, ein Anschlussteil anzugeben, welches einen weiblichen Konusbereich zur Verbindung mit einer Austrageinheit aufweist, und welches derart an einer Austrageinheit mit Sicherungshülse gesichert werden kann, dass das Anschlussteil nicht als Ganzes gegenüber der Austrageinheit verdreht werden muss, selbst wenn die Austrageinheit eine drehfeste Sicherungshülse aufweist. Das Anschlussteil soll ausserdem eine einfache Handhabung sowie eine möglichst einfache Bauweise aufweisen.

Diese Aufgabe wird durch ein Anschlussteil mit den Merkmalen des Anspruchs 1 gelöst. Weitere Ausführungsformen sind in den abhängigen Ansprüchen angegeben.

Die vorliegende Erfindung stellt also ein Anschlussteil mit zumindest einem ersten Kupplungsteil zu Verfügung, mittels welchem das Anschlussteil an eine Austrageinheit, insbesondere eine medizinische Austrageinheit, anschliessbar ist, das erste Kupplungsteil aufweisend
einen weiblichen Konusbereich mit einer durchgehenden Eingangsöffnung, welcher entlang einer Aufschiebrichtung auf einen männlichen Konusbereich der Austrageinheit aufschiebbar ist, um dadurch das Anschlussteil mit der Austrageinheit zu verbinden; und
eine erste Verriegelungsstruktur, welche in einem den weiblichen Konusbereich umgebenden Bereich angeordnet ist und zur Sicherung des Anschlussteils an der Austrageinheit dient.
Dabei weist das erste Kupplungsteil ein Drehelement auf, welches konzentrisch um den weiblichen Konusbereich drehbar ist, und an welchem die erste Verriegelungsstruktur ausgebildet ist. Das Drehelement weist ausserdem ein Betätigungselement auf, welches dazu dient, das Drehelement relativ zum weiblichen Konusbereich zu drehen, selbst wenn die Austrageinheit eine drehfeste Sicherungshülse aufweist.

Eine derartige Ausgestaltung des Anschlussteils erlaubt es, das Anschlussteil mit der Austrageinheit zu verbinden und an dieser zu sichern, ohne dass das Anschlussteil gegenüber der Austrageinheit verdreht werden muss, selbst wenn die Austrageinheit eine drehfeste Sicherungshülse aufweist.

Unter einem weiblichen Konusbereich wird ein Bereich verstanden, welcher eine konisch ausgebildete Innenfläche aufweist, und unter einem männlichen Konusbereich ein Bereich, welcher eine konisch ausgebildete Aussenfläche aufweist. Während sich die Innenfläche des weiblichen Konusbereiches in Aufschiebrichtung aufweitet, verjüngt sich die Aussenfläche des männlichen Konusbereiches in eine entgegen zur Aufschiebrichtung weisende Richtung. Der weibliche bzw. der männliche Konusbereich definiert dabei eine Längsachse, welche sich zentral bezüglich dieser Innen- bzw. Aussenfläche erstreckt. Das Drehelement ist bevorzugt um diese Längsachse des weiblichen Konusbereiches herum drehbar.

Das Anschlussteil kann zur Erfüllung von verschiedensten Funktionen gemäss dem Stand der Technik ausgebildet sein. Insbesondere kann das Anschlussteil als ein Adapter ausgebildet sein, welcher das Anschliessen von beliebigen Zubehörteilen, wie zum Beispiel Mischaufsätzen, Injektionskanülen und Verbindungsschläuchen, an der Austrageinheit ermöglicht. Unter dem Anschliessen eines Anschlussteils an der Austrageinheit oder eines Zubehörteiles am Anschlussteil ist jeweils ein derartiges Anschliessen gemeint, bei dem die beiden betreffenden Teile nicht nur aneinander angebracht sind, sondern auch funktionell zusammenwirken. Ein solches Zusammenwirken ist insbesondere dadurch realisiert, dass eine Fluidleitung, welche in einem Teil ausgebildet ist, in eine im anderen Teil vorhandene Fluidleitung mündet.

Bei der Austrageinheit kann es sich insbesondere um eine handelsübliche Spritze zum Verabreichen eines Medikamentes an Patienten, also um eine Einfachkolbenspritze, handeln.

Das Anschlussteil weist zumindest zwei parallel zueinander angeordnete erste Kupplungsteile auf. Unter einer parallelen Anordnung der ersten Kupplungsteile ist eine derartige Anordnung gemeint, bei welcher die weiblichen Konusbereiche der Kupplungsteile parallel zueinander angeordnet sind, wobei sich insbesondere die Längsachsen dieser Konusbereiche parallel zueinander erstrecken. Das Anschlussteil ist dadurch an eine Mehrzahl von Austrageinheiten, insbesondere mehreren Einfachkolbenspritzen, oder an eine Mehrfachkolbenspritze, insbesondere einer Zweifachkolbenspritze, bzw. an eine Kartusche mit mehreren Reservoirs und entsprechenden Kupplungsteilen anschliessbar. Eine derartige Mehrfachkolbenspritze bzw. Kartusche weist mehrere Reservoirs mit entsprechenden Austrittsöffnungen auf. Die in den verschiedenen Reservoirs enthaltenen Produkte sind einzeln durch einzelne Kolben oder gemeinsam mittels eines Mehrfachkolbens durch die verschiedenen Austrittsöffnungen ausstossbar. Bei einem Mehrfachkolben sind dazu die Kolben an ihren proximalen Enden miteinander verbundenen. Vorteilhaft sind die ersten Kupplungsteile jeweils gleichartig ausgebildet. Sie können jedoch auch unterschiedlich ausgebildet sein und eine Kodierung aufweisen, um nur das Anschliessen von bestimmten Austrageinheiten an jeweils ein erstes betreffendes Kupplungsteil zuzulassen.

In einer bevorzugten Ausführungsform ist das Anschlussteil als ein Anschlussadapter ausgebildet, welcher zum Anschliessen von weiteren Zubehörteilen an die Austrageinheit dient. Ein derartiger Anschlussadapter kann beispielsweise das Verbinden von unterschiedlichen Anschlusssystemen ermöglichen. Ein Zubehörteil mit einem proprietären Kupplungsteil eines ersten Anschlusssystems ist dann mittels des Anschlussadapters an die Austrageinheit anschliessbar, welche ein proprietäres Kupplungsteil gemäss einem zweiten Anschlusssystem aufweist. Bevorzugt weist das Anschlussteil deshalb eine Ausgangsöffnung auf, welche mit der Eingangsöffnung in fluid-kommunizierender Verbindung steht, sowie eine Anschlussstruktur, welche dazu geeignet ist, ein Zubehörteil derart am Anschlussteil anzuschliessen, dass die Ausgangsöffnung fluiddicht in einen gegenüberliegende Fluidleitung des Zubehörteiles mündet. Beim Zubehörteil kann es sich beispielsweise um einen Mischaufsatz, eine Injektionskanüle, eine Sprühdüse, ein Verschlussteil, einen Verbindungsschlauch, eine andere Spritze oder Austrageinheit, oder auch um ein Zubehörteil, welches das Anschliessen von nachmals weiteren Anschlusselementen erlaubt, handeln. Die Anschlussstruktur kann zum Beispiel dazu ausgebildet sein, eine Schraubverbindung, eine Bajonettverbindung oder eine Schnappverbindung mit dem Zubehörteil einzugehen.

Vorzugsweise bildet der weibliche Konusbereich einen Luer-Konus, das heisst einen genormten Konus mit einer Steigung von 6 %.

Vorzugsweise ist die erste Verriegelungsstruktur auf der radialen Aussenseite des Drehelementes angeordnet und insbesondere als ein Aussengewinde ausgebildet. Eine derartige Ausgestaltung der Verriegelungsstruktur kann insbesondere bewirken, dass beim Sichern des Anschlussteils an der Austrageinheit der weibliche Konusbereich des Anschlussteils zum männlichen Konusbereich der Austrageinheit hinbewegt und auf diesen aufgepresst wird. Die Verbindung zwischen Anschlussteil und Austrageinheit und insbesondere die Dichtwirkung dieser Verbindung kann dadurch verbessert werden. Alternativ können als erste Verriegelungsstruktur anstatt eines Aussengewindes zum Beispiel zwei radial gegenüberliegende Nasen vorgesehen sein, die in eine Gewindestruktur oder in Führungsrillen der Sicherungshülse der Austrageinheit eingreifen.

Zum Anbringen des Drehelementes ist im Bereich des weiblichen Konusbereiches eine Stufe ausgebildet mit einer Anschlagfläche, welche in eine zur Aufschiebrichtung im Wesentlichen entgegengesetzte Richtung weist. Vorzugsweise ist die Stufe umlaufend ausgebildet. Der Bereich des weiblichen Konusbereiches umfasst dabei die Aussenfläche des weiblichen Konusbereiches, welche sich radial um die sich in Aufschiebrichtung aufweitende, konische Innenfläche herum erstreckt, sowie die Aussenfläche eines allfälligen Anschlussrohres, welches sich unmittelbar vom weiblichen Konusbereich in die entgegen zur Aufschiebrichtung weisende Richtung weg erstreckt. Das Drehelement weist ein oder mehrere, vorzugsweise genau zwei Hintergreifelemente auf, welche selbst jeweils ebenfalls eine Anschlagfläche aufweisen, mit welcher sie derart an dieser im Bereich des weiblichen Konusbereiches angeordneten Anschlagfläche anschlagen, dass eine weitere Bewegung des Drehelementes in Aufschiebrichtung verhindert ist. Vorzugsweise bilden jeweils zwei der Hintergreifelemente eines Drehelements ein sich diametral gegenüberliegendes Paar. Um die Montage zu erleichtern, sind die Hintergreifelemente flexibel ausgebildet und weisen vorteilhaft jeweils ein freies Ende auf. Am freien Ende ist dabei jeweils bevorzugt eine Schrägfläche ausgebildet, welche in Bezug auf die Längsachse des weiblichen Konusbereiches geneigt ist. Bevorzugt ist die Schrägfläche dabei derart geneigt, dass sie sich in die entgegen zur Aufschiebrichtung weisende Richtung von der Längsachse des weiblichen Konusbereiches entfernt.

Das Drehelement ist bevorzugt frei drehbar. Damit ist gemeint, dass das Drehelement um einen Winkelbereich von mindestens 360° drehbar ist.

Bevorzugt weist das Drehelement eine Drehhülse auf, welche den weiblichen Konusbereich im Wesentlichen umlaufend umgibt und an deren Aussenseite die Verriegelungsstruktur ausgebildet ist. Das Betätigungselement ist zudem bevorzugt als ein Drehring ausgebildet, welcher an der Drehhülse angebracht ist. Der Drehring weist dabei vorteilhaft einen grösseren Aussenradius auf als die Drehhülse, wodurch er für den Benutzer besser zugänglich ist. Falls Hintergreifelement vorhanden sind, sind diese vorteilhaft radial im Innern des Drehringes angeordnet.

Es wird ausserdem eine Austragvorrichtung, insbesondere eine medizinische Austragvorrichtung, angegeben, welche ein erfindungsgemässes Anschlussteil sowie eine Austrageinheit aufweist. Die Austrageinheit weist dabei auf
ein Gehäuse mit mindestens einem Reservoir; sowie
zumindest ein zweites Kupplungsteil mit einer Sicherungshülse und mit einem männlichen Konusbereich, welcher radial beabstandet innerhalb der Sicherungshülse angeordnet ist und eine durchgehende Austrittsöffnung aufweist, welche mit dem Reservoir in fluid-kommunizierender Verbindung steht.

Der weibliche Konusbereich des Anschlussteils ist dabei auf den männlichen Konusbereich der Austrageinheit aufschiebbar, so dass das Reservoir mit der Eingangsöffnung in fluid-kommunizierender Verbindung steht. Ausserdem weist die Sicherungshülse eine zweite Verriegelungsstruktur auf, welche derart mit der ersten Verriegelungsstruktur verriegelbar ist, dass das Anschlussteil an der Austrageinheit gesichert ist.

Das zweite Kupplungsteil ist somit komplementär zum ersten Kupplungsteil des Anschlussteils ausgebildet.

Die Sicherungshülse kann drehbar am Gehäuse angebracht sein oder, wie es hier bevorzugt ist, drehfest am Gehäuse angebracht sein. Die zweite Verriegelungsstruktur ist in der Regel innen an der Sicherungshülse angeordnet und bevorzugt als ein Innengewinde ausgebildet. Die zweite Verriegelungsstruktur kann aber zum Beispiel auch als eine Führungsbahn in Form einer Vertiefung auf der Innenseite der Sicherungshülse oder in Form eines durchgehenden Schlitzes in der Sicherungshülse ausgestaltet sein.

Die Austrageinheit kann als eine Einfachkolbenspritze oder auch als eine Doppel- oder Mehrfachkolbenspritze bzw. als eine Kartusche ausgebildet sein. Die Austrageinheit kann in diesem Fall zwei oder mehr parallel zueinander angeordnete zweite Kupplungsteile aufweisen, die dann mit zumindest zwei parallel zueinander angeordneten ersten Kupplungsteilen des Anschlussteils koppel- und verriegelbar sind.

In einer bevorzugten Ausführungsform weist die Doppel- oder Mehrfachkolbenspritze zwei oder mehrere voneinander lösbare, separat voneinander verwendbare Einfachkolbenspritzen auf. Die Einfachkolbenspritzen können dabei beispielsweise an ihren proximalen Enden lösbar mittels eines Verbindungselementes miteinander verbunden sein. Es sind dabei insbesondere handelsübliche Standardspritzen verwendbar, wodurch Mehrfachkolbenspritzen auf eine sehr kostengünstige Weise herstellbar sind. Dadurch, dass die Einfachkolbenspritzen jeweils nicht als Ganzes relativ zum Anschlussteil verdreht werden müssen, kommen sich nach aussen ragende Elemente der Spritzen beim Anschliessen der Spritzen am Anschlussteil nicht in die Quere. Die Spritzen können dadurch näher zueinander angeordnet werden. Der Platzbedarf der Austragvorrichtung ist somit deutlich verringert. Falls das Anschlussteil beispielsweise als ein Mischelement oder als ein Adapter zum Anschliessen von einem Mischaufsatz oder von einem anderen Zubehörteil ausgebildet ist, weisen aufgrund der näheren Anordnung der Spritzen im Anschlussteil vorhandene Fluidkanäle eine insgesamt verkürzte Länge auf. Ein Verlustvolumen des fluiden Produkts, welches nach dem Austragen im Anschlussteil verbleibt, ist dadurch deutlich verringert.

Die Austragvorrichtung kann zudem einen Mischaufsatz mit einer zweiten Anschlussstruktur und mit einer Mischkammer aufweisen, wobei der Mischaufsatz durch Verbinden der ersten Anschlussstruktur mit der zweiten Anschlussstruktur derart am Anschlussteil anschliessbar ist, dass die Ausgangsöffnungen des Anschlussteils jeweils mit der Mischkammer in fluid-kommunizierender Verbindung stehen.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Ansicht einer Austragvorrichtung mit einem erfindungsgemässen Anschlussteil und einer Austrageinheit gemäss einer ersten Ausführungsform;
- Fig. 2: eine perspektivische Detailansicht des in Figur 1 markierten Bereiches der Austragvorrichtung;
- Fig. 3: eine zentrale Schnittansicht in der Ebene III-III der in Figur 1 gezeigten Austragvorrichtung;
- Fig. 4: eine Detailschnittansicht des in Figur 3 markierten Bereiches der Austragvorrichtung;
- Fig. 5: eine perspektivische Ansicht einer Austragvorrichtung mit einem erfindungsgemässen Anschlussteil und einer Austrageinheit gemäss einer zweiten Ausführungsform;
- Fig. 6: eine Detailansicht des in Figur 5 markierten Bereiches der Austragvorrichtung;
- Fig. 7: eine zentrale Schnittansicht in der Ebene VII-VII der in Figur 5 gezeigten Austragvorrichtung; sowie
- Fig. 8: eine Detailschnittansicht des in Figur 7 markierten Bereiches der Austragvorrichtung.

### BESCHREIBUNG BEVORZUGTER AUSFÜHRUNGSFORMEN

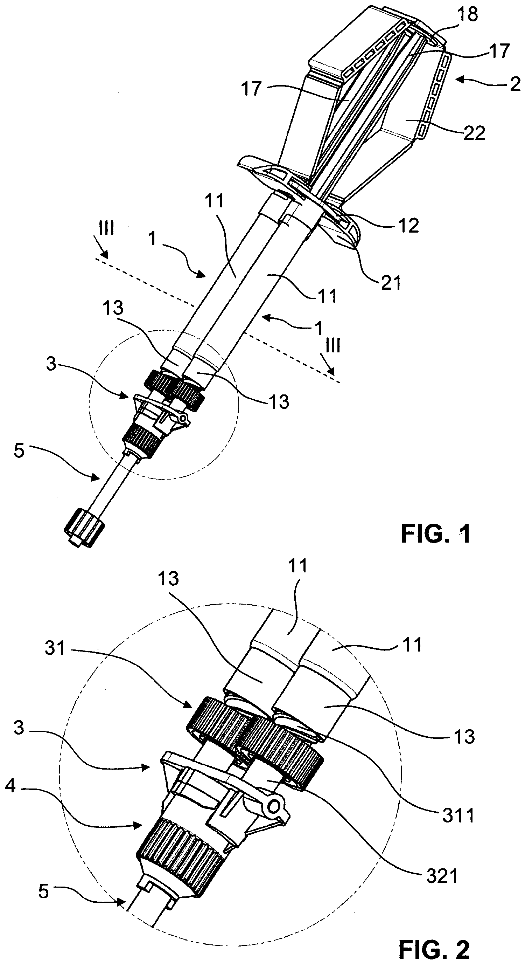

Die Figuren 1 bis 4 zeigen eine medizinische Austragvorrichtung mit einem erfindungsgemässen Anschlussteil 3 gemäss einer ersten Ausführungsform. Das Anschlussteil 3 ist hier als ein Anschlussadapter ausgebildet. Die Austragvorrichtung weist eine Austrageinheit in Form von zwei Spritzen 1 auf, welche jeweils an einem distalen Gehäuseende über ein Anschlussteil 3 miteinander verbunden sind. An ihrem proximalen Ende sind die beiden Spritzen 1 über einen Spritzenhalter 2 lösbar miteinander verbunden. Auf der den Spritzen 1 gegenüberliegenden Seite des Anschlussteils 3 ist ein Mischaufsatz 5 am Anschlussteil 3 angeschlossen und mittels einer Verbindungshülse 4 an diesem gesichert.

Bei den Spritzen 1 handelt es sich in der vorliegenden Ausführungsform jeweils um handelsübliche Spritzen mit einem Gehäuse, welches eine distale Austrittsöffnung 15 sowie eine proximale Öffnung zum Einschieben eines Kolbens 17 aufweist. Das Gehäuse weist ein Reservoir 16 auf, welches zur Aufnahme eines fluiden medizinischen Produktes dient und von einer hohlzylindrischen Seitenwand 11 in eine radiale Richtung begrenzt ist. Durch Vorschieben des Kolbens 17 in das Reservoir 16 hinein ist das fluide Produkt in distaler Richtung durch die Austrittsöffnung 15 ausstossbar. Ein am distalen Ende des Kolbens 17 ausgebildetes Dichtelement 19 dient dabei dazu, ein Entweichen des im Reservoir 16 gelagerten fluiden Produktes in die proximale Richtung zu verhindern. Um das Vorschieben des Kolbens 17 in das Reservoir 16 hinein zu erleichtern, ist eine Druckplatte 18 am proximalen Ende des Kolbens 17 ausgebildet. Ausserdem weist das Spritzengehäuse Halteflügel 12 auf, welche am proximalen Ende der Seitenwand 11 angebracht sind. Die Halteflügel 12 stehen dabei in zwei diametral gegenüberliegende Richtungen vom Gehäuse nach aussen hin ab und dienen dazu, dem Benutzer eine in distale Richtung weisende Auflagefläche für die Finger anzubieten, während der Kolben 17 durch Daumendruck auf die Druckplatte 18 vorgeschoben wird. Im Bereich der Austrittsöffnung 15 ist an der Spritze 1 ein Kupplungsteil ausgebildet, welches ein Anbringen von beliebigen Zubehörteilen an der Spritze 1 erlaubt, wie beispielsweise Injektionskanülen, Sprühdüsen, Verschlüsse, Verbindungsschläuche etc.

Das Kupplungsteil der Spritze 1 weist einen männlichen Konusbereich in Form eines Luer-Konus 14 auf. Der männliche Luer-Konus 14, welcher die Austrittsöffnung 15 begrenzt, verjüngt sich auf seiner Aussenseite in eine vom Reservoir 16 weg weisende Richtung. Um den männlichen Luer-Konus 14 herum und beabstandet dazu ist eine Sicherungshülse 13 fest am Spritzengehäuse angebracht. Die Sicherungshülse 13 weist auf ihrer Innenseite eine Verriegelungsstruktur in Form eines Innengewindes auf.

In der vorliegenden Ausführungsform sind die beiden Spritzen 1 jeweils an ihrem proximalen Gehäuseende, im Bereich der Halteflügel 12, mittels eines Spritzenhalters 2 lösbar miteinander verbunden. Der Spritzenhalter 2 weist dazu ein Verbindungselement 21 auf, welches die Halteflügel 12 derart aufnimmt, dass die einander diametral gegenüberliegenden Halteflügel 12 der einen Spritze 1 parallel zu denjenigen der anderen Spritze 1 angeordnet sind. Der Spritzenhalter 2 weist ausserdem ein Betätigungselement 22 auf, durch welches die Kolben 17 der beiden Spritzen 1 gemeinsam vorschiebbar sind. Der Spritzenhalter 2 ist insbesondere in der WO 2010/009563 auf der Seite 2, Zeile 22, bis Seite 4, Zeile 17, im Detail beschrieben, worauf hier ausdrücklich Bezug genommen wird.

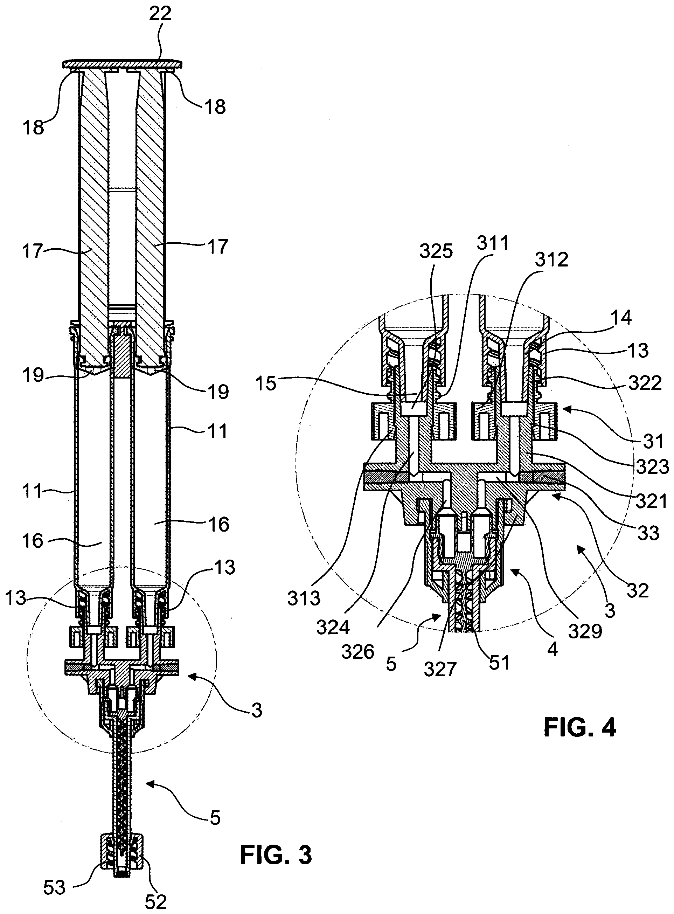

Das Anschlussteil 3, welches insbesondere in der Figur 4 im Detail gezeigt ist, weist in der vorliegenden Ausführungsform ein Gehäuse 32 auf, an welchem zwei Drehelemente 31 angebracht sind. Die beiden Drehelemente 31 gehören zu jeweils einem von insgesamt zwei am Anschlussteil 3 vorhandenen Kupplungsteilen, welche identisch zueinander ausgebildet sind. Jedes der Kupplungsteile dient dazu, das Anschlussteil 3 mit jeweils einer Spritze 1 zu verbinden und an dieser zu sichern. Die beiden Kupplungsteile erstrecken sich dabei parallel zueinander und weisen jeweils einen weiblichen Konusbereich 322 mit einer durchgehenden Eingangsöffnung 325 auf. Beim weiblichen Konusbereich 322 handelt es sich dabei um einen weiblichen Luer-Konus, welcher dazu geeignet ist, entlang einer Aufschiebrichtung auf den an der Spritze 1 ausgebildeten männlichen Luer-Konus 14 aufgeschoben zu werden, um dadurch eine Verbindung zwischen dem Anschlussteil 3 und der Spritze 1 herzustellen. Die Spritze 1 und das Anschlussteil 3 sind dann derart miteinander verbunden, dass die Austrittsöffnung 15 der Spritze 1 nach aussen hin fluiddicht in die Eingangsöffnung 325 des Anschlussteils 3 mündet. Die Eingangsöffnung 325 weitet sich dazu in Aufschiebrichtung hin auf. In die zur Aufschiebrichtung entgegengesetzte Richtung geht der weibliche Luer-Konus 322 in ein Anschlussrohr 321 über. Das Anschlussrohr 321 weist eine Längsöffnung 324 auf, in welche die Eingangsöffnung 325 mündet. Die Längsöffnung bildet dabei eine Fluidleitung. Das Anschlussrohr 321 weist eine dickere Wandstärke als der Luer-Konus 322 auf. Im Übergangsbereich zwischen Eingangsöffnung 325 und Fluidleitung 324 ist deswegen auf einer Innenseite des Gehäuses 32 eine umlaufende Stufe ausgebildet. Ausserdem ist auf der Aussenseite des Anschlussrohres 321, im Bereich des weiblichen Konusbereiches 322, eine umlaufende Stufe 323 ausgebildet. Die Stufe 323 weist insbesondere eine Anschlagfläche auf, welche in eine entgegensetzt zur Aufschiebrichtung gerichtete Richtung weist.

Die Kupplungsteile des Anschlussteils 3 weisen zudem jeweils ein frei drehbares Drehelement 31 auf. Das Drehelement 31 umfasst unter anderem eine Gewindehülse 311, welche derart dimensioniert ist, dass ihr Innenradius geringfügig grösser als der Aussenradius des weiblichen Luer-Konus 322 ist. Auf der radialen Aussenseite der Gewindehülse 311 ist eine Verriegelungsstruktur in Form eines Aussengewindes ausgebildet. Dieses Aussengewinde ist somit um den weiblichen Luer-Konus 322 herum angeordnet

Am distalen Ende der Gewindehülse 311 ist über einen sich in radialer Richtung senkrecht nach aussen hin erstreckenden Verbindungsbereich ein Drehring 312 angebracht. Der Drehring 312 weist dabei einen wesentlich grösseren Aussenradius als die Gewindehülse 311 auf. Im radialen Innenbereich des Drehringes 312 sind Hintergreifelemente 313 ausgebildet, welche sich jeweils in distaler Richtung erstrecken und eine in proximale Richtung weisende Anschlagfläche aufweisen. Die Hintergreifelemente 313 erstrecken sich dabei gleich weit in die distale Richtung wie der Drehring 312. Als mit den Hintergreifelementen 313 zusammenwirkendes Element dient die am Gehäuse 32 ausgebildete Stufe 323. Aufgrund des Anschlages der Hintergreifelemente 313 an der in distaler Richtung weisenden Anschlagfläche der Stufe 323 begrenzt die Stufe 323 eine Bewegung des Drehelementes 31 relativ zum weiblichen konischen Bereich 322 in die proximale Richtung. Das Drehelement 31 ist dabei jedoch frei drehbar, d.h. um einen Winkelbereich von mindestens 360°, um den weiblichen Luer-Konus 322 herum angeordnet. Die Hintergreifelemente 313 sind in dieser Ausführungsform flexibel ausgebildet, um bei der Montage ein erleichtertes Anbringen der Drehelemente 31 am Gehäuse 32 zu ermöglichen.

Die Drehelemente 31 dienen insgesamt jeweils dazu, das mit den Spritzen 1 über die Konusbereiche 322 und 14 verbundene Anschlussteil 3 an den Kupplungsteilen der Spritzen 1 zu sichern. Dazu wird das Aussengewinde der Gewindehülse 311 in das Innengewinde der Sicherungshülse 13 eingeschraubt, ohne dass das Adaptergehäuse 32 gegenüber dem Spritzengehäuse verdreht werden muss. Dadurch, dass es nicht notwendig ist, die beiden Spritzengehäuse gegenüber dem Adaptergehäuse 32 zu verdrehen, ist eine viel einfachere Anbringung der die beiden Spritzengehäuse am Anschlussteil sowie eine nähere Anordnung der beiden Spritzen 1 ermöglicht. Wenn das Anschlussteil 3 Kupplungsteile gemäss dem Stand der Technik aufweisen würde, müssten die Spritzen als Ganzes jeweils in das Anschlussteil 3 eingeschraubt werden, was nur bei einem derartig genügend grossen Abstand der beiden Spritzen 1 möglich wäre, dass die Haltflügel 12 nicht gegenseitig aneinander anstossen.

Der Drehring 312 gewährleistet dem Benutzer die Zugänglichkeit des Drehelementes 31. Beim Einschrauben des Drehelementes 31 in die Sicherungshülse 13 wird der männliche Luer-Konus 14 weiter in den weiblichen Luer-Konus 322 hineingeschoben. Aufgrund der konischen Formen der Luer-Konusse 14 und 322 wird der weibliche Luer-Konus 322 beim Einschrauben der Gewindehülse 311 zunehmend gegen den männlichen Luer-Konus 14 gepresst. Die fluiddichte Verbindung zwischen dem männlichen Luer-Konus 14 und dem weiblichen Luer-Konus 322 wird dadurch beim Einschrauben des Drehelementes 31 in die Sicherungshülse 13 verbessert. Zudem dient die Verschraubung der mechanischen Sicherung der Luer-Verbindung.

Die beiden parallelen Anschlussrohre 321 sowie die von diesen begrenzten Fluidleitungen 324 des Gehäuses 32 erstrecken sich jeweils parallel zueinander in die distale Richtung. Die Fluidleitungen 324 münden an ihrem distalen Ende jeweils in eine Querleitung 329. Diese beiden Querleitungen 329 erstrecken sich dabei derart in eine senkrecht zu den Fluidleitungen 324 stehende Richtung, dass sie sich einander annähern. Die herstellungsbedingten, voneinander weg gewandten Seitenöffnungen der Querleitungen 329 sind jeweils durch einen Pfropfen 33 fluiddicht verschlossen. Die Querleitungen 329 münden dann jeweils in senkrecht dazu stehende, sich in distale Richtung hin erstreckende Anschlussleitungen, welche schliesslich in distale Ausgangsöffnungen 326 münden. Die beiden Ausgangsöffnungen 326 sind somit näher zueinander angeordnet als die Eingangsöffnungen 325.

Im Bereich der Ausgangsöffnungen 326 ist eine Anschlussstruktur 327 ausgebildet, welche zum Anschliessen von unterschiedlichsten Zubehörteilen dient. Bei einem solchen Zubehörteil kann es sich beispielsweise um einen Mischaufsatz 5 handeln. Dieser ist, wie in Figur 4 gezeigt ist, über eine Verbindungshülse 4 mittels Bajonettverschluss an der Anschlussstruktur 327 des Anschlussteils 3 gesichert. Die Ausgangsöffnungen 326 des Anschlussteils 3 münden dabei in entsprechende, gegenüberliegende Eingangskanäle des Mischaufsatz 5. Diese Eingangskanäle des Mischaufsatzes 5 sind in einer Mischkammer 51 zusammengeführt. Die Mischkammer 51 weist einen Mischwendel auf, um die beiden fluiden Produkte der beiden Spritzen 1 miteinander zu vermischen. Am distalen Ende des Mischaufsatzes 5 ist ein Kupplungsteil bestehend aus einer drehbaren Schraubhülse 52 und einem männlichen Luer-Konus 53 ausgebildet. Dieses distal angeordnete Kupplungsteil des Mischaufsatzes 5 dient zum Anschliessen von weiteren Anschlusselementen, wie beispielsweise einer Injektionskanüle oder einem Verbindungsschlauch. Der Mischaufsatz ist insbesondere im Dokument US 2001/0004082 detailliert beschrieben, auf welches hier Bezug genommen wird.

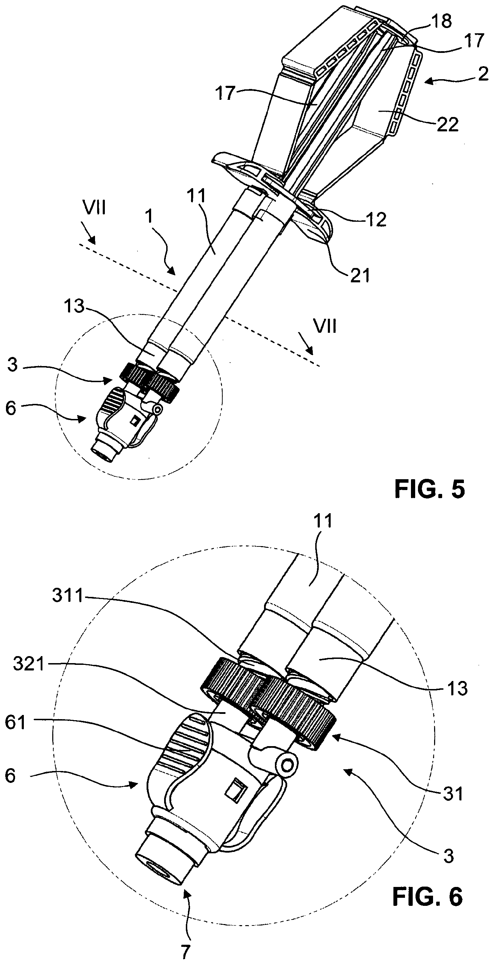

Eine zweite Ausführungsform einer Austragvorrichtung mit einem erfindungsgemässen, als Anschlussadapter ausgebildeten Anschlussteil ist in den Figuren 5 bis 8 gezeigt. Gleiche oder ähnliche Teile der Austragvorrichtung sind dabei mit denselben Bezugszeichen wie bei der ersten Ausführungsform gekennzeichnet. Anders als im ersten Ausführungsbeispiel ist ein Zubehörteil hier nicht mittels einer Bajonettverbindung am Anschlussteil 3 anbringbar, sondern das Zubehörteil ist als ein Aufsteckelement 6 ausgebildet, welches auf die Anschlussstruktur 327 aufsteckbar ist und dabei eine Schnappverbindung mit dieser eingeht. Dazu weist die Anschlussstruktur 327 zwei voneinander weg gewandte, nach aussen hin ragende Vorsprünge auf, welche mit am Aufsteckelement 6 ausgebildeten Hintergreifelementen 62 in Verbindung treten. Mittels Eindrücken zweier einander gegenüberliegender Druckgriffe 61 sind die Hintergreifelemente 62 des Aufsteckelementes 6 nach aussen hin voneinander weg drückbar, so dass das Aufsteckelement 6 in einfacher Weise vom Anschlussteil 3 trennbar ist. Mögliche konkrete Ausgestaltungen der Anschlussstruktur 327 sind insbesondere im Dokument WO 2007/109915 detailliert beschrieben und dort mit den Bezugszeichen 6, 43, 37, 120 und 220 versehen. Eine genaue Beschreibung des Aufsteckelements 6 findet sich im Dokument WO 2010/020061, wobei das Aufsteckelement 6 dort als ein Anschlussstück beschrieben ist. Das Aufsteckelement 6 ist hier mit einer Spraydüse 7 versehen. Es ist jedoch das Anbringen von beliebigen anderen Anschlusselementen am Aufsteckelement 6 denkbar, wie z. B. von Verschlussdeckeln, Mischelementen, Injektionskanülen etc.

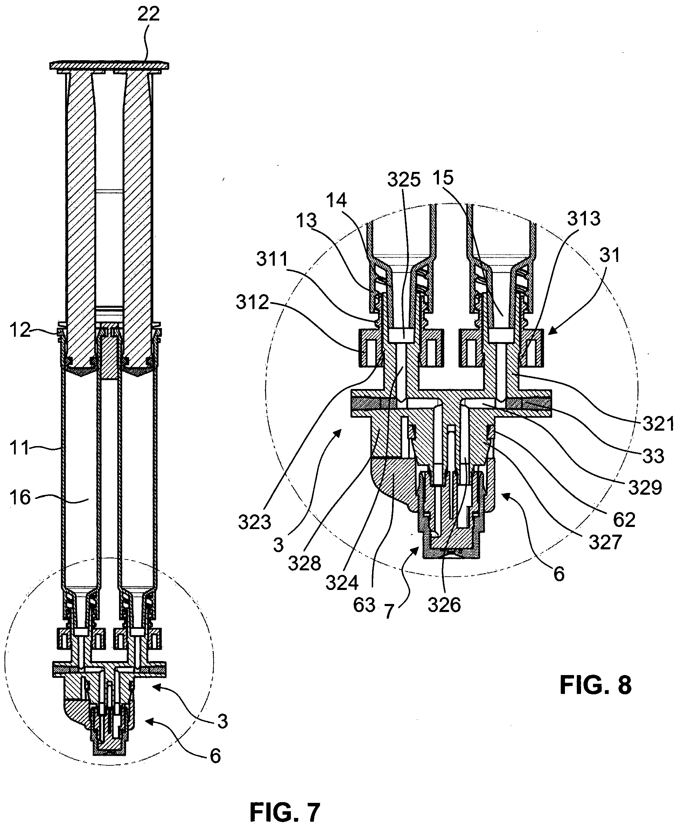

Je nach Funktion und Ausgestaltung des Zubehörteiles kann es erforderlich sein, dass die Ausgangsöffnungen 326 des Anschlussteils 3 in jeweils genau eine zugeordnete Eingangsleitung des Zubehörteiles münden. Das Anschlussteil 3 weist deshalb in dieser Ausführungsform zusätzlich ein Positionierungsprofil 328 auf, welches den Benutzer bei der korrekten Anbringung des Aufsteckelementes 6 am Anschlussteil 3 unterstützt. Der Benutzer muss dabei das Aufsteckelement 6 derart am Anschlussteil 3 anbringen, dass ein einseitig vom Aufsteckelement 6 abragender Positionierungsflügel 63 direkt gegenüberliegend zum Positionierungselement 328 platziert ist (siehe Figur 8). Ein korrektes Anbringen des Aufsteckelementes 6 am Anschlussteil 3 kann ausserdem dadurch sichergestellt sein, dass die Anschlussstruktur 327 sowie die entsprechend komplementäre Anschlussstruktur des Aufsteckelementes 6 asymmetrisch in Bezug auf die Positionen der beiden Ausgangsöffnungen 326 ausgebildet sind. Das Zubehörteil ist dann nur auf eine mögliche Weise am Anschlussteil 3 anschliessbar.

Das Spritzengehäuse, der Kolben 17, der Spritzenhalter 2, das Gehäuse 32 des Anschlussteils 3, das Drehelement 31, die Verbindungshülse 4, das Aufsteckelement 6 sowie die Spraydüse 7 sind hier jeweils als Ganzes einstückig ausgebildet und aus einem Kunststoff in einem Spritzgussverfahren hergestellt. Es ist jedoch auch denkbar, das jeweilige Zubehörteil, wie beispielsweise den Mischaufsatz 5 oder das Aufsteckelement 6, direkt einstückig mit dem Gehäuse 32 des Anschlussteils 3 auszubilden. Auch der Spritzenhalter könnte direkt einstückig an zwei oder mehreren Spritzengehäusen angebracht sein.

Die Erfindung ist selbstverständlich nicht auf die vorstehenden Ausführungsbeispiele beschränkt, und eine Vielzahl von Abwandlungen ist möglich. So kann das Anschlussteil, welches nicht als ein Anschlussadapter ausgebildet sein muss, beispielsweise nur ein Kupplungsteil umfassen, welches zum Anschliessen von nur einer Spritze 1 am Anschlussteil dient. Es können jedoch auch mehr als zwei Kupplungsteile vorgesehen sein. Die Austrageinheit muss zudem nicht als eine wie in den Ausführungsbeispielen beschriebene Spritze ausgestaltet sein. Eine beliebige Ausgestaltung der Austrageinheit gemäss dem Stand der Technik ist hierzu denkbar.

Die am Drehelement 31 und der Spritze 1 ausgebildeten Verriegelungsstrukturen müssen nicht zwingend als ein Aussen- bzw. ein Innengewinde ausgebildet sein, sondern können auf beliebige Weise gemäss dem Stand der Technik ausgestaltet sein. Es wäre auch möglich, am Drehelement 31 lediglich nach aussen hin vorstehende, z.B. diametral gegenüberliegende Nasen vorzusehen, welche in ein Gewinde oder eine andere Führungsstruktur der Sicherungshülse eingreifen. Insbesondere ist beispielsweise die Verwendung eines Bajonettverschlusses denkbar. Die Sicherungshülse 13 könnte ausserdem frei drehbar am Spritzengehäuse 11 angebracht sein und für das Aufschrauben eine Blockierfunktion, wie in der US 2006/0157984 beschrieben, aufweisen. Ein schleichendes Lösen der Verriegelung zwischen Anschlussteil und Spritze, beispielsweise bei Vibrationen, wäre dadurch verhindert.

Weiters könnte das Betätigungselement des Drehelementes 31 anstatt durch einen Drehring beispielsweise durch nach aussen hin vorstehende Vorsprünge ausgestaltet sein. Der Drehring könnte auch derart an der Gewindehülse 311 angebracht sein, dass sich die Gewindehülse beabstandet dazu radial im Innern des Drehringes befindet. Bezüglich der Zubehörteile 5 bzw. 6 ist eine Vielzahl von anderen Zubehörteilen denkbar, welche zur Erfüllung von verschiedenen Funktionen ausgebildet sein können. Im Stand der Technik findet sich hierzu eine Vielzahl von Beispielen.

### BEZUGSZEICHENLISTE

| | | | |
|---|---|---|---|
| 1 | Spritze | 323 | Stufe |
| 11 | Gehäuseseitenwand | 324 | Fluidleitung |
| 12 | Halteflügel | 325 | Eingangsöffnung |
| 13 | Sicherungshülse | 326 | Ausgangsöffnung |
| 14 | Männlicher Luer-Konus | 327 | Anschlussstruktur |
| 15 | Austrittsöffnung | 328 | Positionierungsprofil |
| 16 | Reservoir | 329 | Querleitung |
| 17 | Kolben | 33 | Pfropfen |
| 18 | Druckplatte | | |
| 19 | Dichtelement | 4 | Verbindungshülse |
| | | | |
| 2 | Spritzenhalter | 5 | Mischaufsatz |
| 21 | Verbindungselement | 51 | Mischkammer |
| 22 | Betätigungselement | 52 | Schraubhülse |
| | | 53 | Luer-Innenkonus |
| 3 | Anschlussteil | | |
| 31 | Drehelement | 6 | Aufsteckelement |
| 311 | Gewindehülse | 61 | Druckgriff |
| 312 | Drehring | 62 | Hintergreifelement |
| 313 | Hintergreifelement | 63 | Positionierungsflügel |
| 32 | Gehäuse | | |
| 321 | Anschlussrohr | 7 | Spraydüse |
| 322 | Weiblicher Luer-Konus | | |

## Patentansprüche

1. Anschlussteil (3) mit zumindest zwei parallel zueinander angeordneten ersten Kupplungsteilen, mittels welchen das Anschlussteil (3) an eine Austrageinheit (1) oder an eine Mehrzahl von Austrageinheiten (1) anschliessbar ist, wobei die zumindest zwei ersten Kupplungsteile jeweils aufweisen
einen weiblichen Konusbereich (322) mit einer durchgehenden Eingangsöffnung (325), welcher entlang einer Aufschiebrichtung auf einen männlichen Konusbereich (14) der Austrageinheit (1) oder einer der Austrageinheiten (1) aufschiebbar ist, um dadurch das Anschlussteil (3) mit der Austrageinheit (1) oder einer der Austrageinheiten (1) zu verbinden, und
ein Drehelement (31), welches konzentrisch um den weiblichen Konusbereich (322) drehbar ist und eine Drehhülse (311) aufweist, an deren Aussenseite eine erste Verriegelungsstruktur ausgebildet ist, welche in einem den weiblichen Konusbereich (322) radial umgebenden Bereich angeordnet ist und zur Sicherung des Anschlussteils (3) an der Austrageinheit (1) oder an einer der Austrageinheiten (1) dient,
wobei die Drehelemente (31) der zumindest zwei ersten Kupplungsteile jeweils ein als Drehring (312) ausgebildetes und an der Drehhülse (311) angebrachtes Betätigungselement aufweisen, welches dazu dient, das Drehelement (31) relativ zum weiblichen Konusbereich (322) zu drehen,
wobei im Bereich der weiblichen Konusbereiche (322) der zumindest zwei ersten Kupplungsteile jeweils eine Stufe (323) ausgebildet ist mit einer ersten Anschlagfläche, welche in eine zur Aufschiebrichtung im Wesentlichen entgegengesetzte Richtung weist,
und wobei die Drehelemente (31) der zumindest zwei ersten Kupplungsteile jeweils zumindest ein flexibel ausgebildetes Hintergreifelement (313) aufweisen, welches radial im Innern des Drehrings (312) angeordnet ist und sich in eine zur Aufschiebrichtung im Wesentlichen entgegengesetzte Richtung erstreckt, und welches eine zweite, in Aufschiebrichtung weisende Anschlagfläche aufweist und mit dieser zweiten Anschlagfläche derart an der ersten Anschlagfläche anschlägt, dass die erste Anschlagfläche eine weitere Bewegung des Drehelementes (31) in Aufschiebrichtung verhindert.

2. Anschlussteil (3) gemäss einem der vorhergehenden Ansprüche, wobei die Drehelemente (31) jeweils entlang der Aufschiebrichtung relativ zum weiblichen Konusbereich (322) bewegbar sind.

3. Anschlussteil (3) gemäss einem der Ansprüche 1 oder 2, wobei den zumindest zwei ersten Kupplungsteilen jeweils eine Ausgangsöffnung (326) zugeordnet ist, welche mit der jeweiligen Eingangsöffnung (325) in fluid-kommunizierender Verbindung steht, und wobei das Anschlussteil (3) eine erste Anschlussstruktur (327) aufweist, welche dazu geeignet ist, ein Zubehörteil (5; 6) derart am Anschlussteil (3) anzuschliessen, dass die Ausgangsöffnungen (326) fluiddicht in eine gegenüberliegenden Fluidleitung des Zubehörteiles (5; 6) münden.

4. Anschlussteil (3) gemäss einem der vorhergehenden Ansprüche, wobei die weiblichen Konusbereiche (322) jeweils einen Luer-Konus bilden.

5. Anschlussteil (3) gemäss einem der vorhergehenden Ansprüche, wobei die ersten Verriegelungsstrukturen jeweils als ein Aussengewinde ausgebildet sind.

6. Anschlussteil (3) gemäss einem der vorhergehenden Ansprüche, wobei die Drehringe (312) jeweils über einen sich in radialer Richtung senkrecht nach aussen hin erstreckenden Verbindungsbereich an der Drehhülse (311) angebracht sind.

7. Anschlussteil (3) gemäss einem der vorhergehenden Ansprüche, wobei die Drehringe (312) jeweils einen grösseren Aussenradius aufweisen als die jeweilige Drehhülse (311).

8. Anschlussteil (3) gemäss einem der vorhergehenden Ansprüche, wobei die weiblichen Konusbereiche (322) jeweils eine konisch ausgebildete Innenfläche aufweisen und eine Längsachse definieren, welche sich zentral bezüglich dieser Innenfläche erstreckt, und wobei am freien Ende der Hintergreifelemente (313) jeweils eine Schrägfläche ausgebildet ist, welche derart in Bezug auf die Längsachse des jweiligen weiblichen Konusbereiches (322) geneigt ist, dass sich die Schrägfläche in die entgegen zur Aufschiebrichtung weisende Richtung von der Längsachse des weiblichen Konusbereiches (322) entfernt.

9. Austragvorrichtung aufweisend ein Anschlussteil (3) gemäss einem der vorhergehenden Ansprüche, sowie eine Austrageinheit (1) oder eine Mehrzahl von Austrageinheiten (1), welche jeweils aufweisen
ein Gehäuse mit mindestens einem Reservoir (16), und
zumindest ein zweites Kupplungsteil mit einer Sicherungshülse (13) und mit einem männlichen Konusbereich (14), welcher radial beabstandet innerhalb der Sicherungshülse (13) angeordnet ist und eine durchgehende Austrittsöffnung (15) aufweist, welche mit dem Reservoir (16) in fluid-kommunizierender Verbindung steht,
wobei die weiblichen Konusbereiche (322) des Anschlussteils (3) jeweils auf einen der männlichen Konusbereiche (14) der Austrageinheit (1) bzw. der Austrageinheiten (1) aufschiebbar sind, so dass die Reservoirs (16) jeweils mit einer der Eingangsöffnungen (325) in fluid-kommunizierender Verbindung stehen,
und wobei die Sicherungshülsen (13) jeweils eine zweite Verriegelungsstruktur aufweisen, welche derart mit einer der ersten Verriegelungsstrukturen verriegelbar ist, dass das Anschlussteil (3) an der Austrageinheit (1) bzw. an einer der Austrageinheiten (1) gesichert ist.

10. Austragvorrichtung gemäss Anspruch 9, wobei die Sicherungshülsen (13) jeweils drehfest am entsprechenden Gehäuse angebracht sind.

11. Austragvorrichtung gemäss einem der Ansprüche 9 oder 10, aufweisend eine Austrageinheit (1), welche als eine Doppel- oder Mehrfachkolbenspritze mit zumindest zwei parallel angeordneten zweiten Kupplungsteilen ausgebildet ist, und wobei das Anschlussteil (3) zumindest zwei parallel zueinander angeordnete erste Kupplungsteile aufweist, wobei jeder dieser ersten Kupplungsteile mit jeweils einem der zweiten Kupplungsteile der Austrageinheit (1) koppel- und verriegelbar sind.

12. Austragvorrichtung gemäss Anspruch 11, wobei die Doppel- oder Mehrfachkolbenspritze zwei oder mehrere voneinander lösbare, separat voneinander verwendbare Einfachkolbenspritzen (1) aufweist.

13. Austragvorrichtung gemäss einem der Ansprüche 11 oder 12, wobei das Anschlussteil (3) eine erste Anschlussstruktur und mehrere Ausgangsöffnungen (326) aufweist, welche jeweils mit einer der Eingangsöffnungen (325) in fluid-kommunizierender Verbindung stehen, wobei ausserdem die Austragvorrichtung einen Mischaufsatz (5) mit einer zweiten Anschlussstruktur und mit einer Mischkammer (51) aufweist, und wobei der Mischaufsatz (5) durch Verbinden der ersten Anschlussstruktur (327) mit der zweiten Anschlussstruktur derart am Anschlussteil (3) anschliessbar ist, dass die Ausgangsöffnungen (326) des Anschlussteils (3) jeweils mit der Mischkammer (51) in fluid-kommunizierender Verbindung stehen.

## Claims

1. An attachment part (3) with at least two first coupling parts arranged parallel to one another, by means of which the attachment part (3) is able to be attached to a dispensing unit (1) or to a plurality of dispensing units (1), wherein the at least two first coupling parts each have
a female cone area (322), which has an inlet opening (325) going throughout the female cone area (322), and which is able to be pushed on along a pushing-on direction onto a male cone area (14) of the dispensing unit (1) or of one of the dispensing units (1), in order to thereby connect the attachment part (3) with the dispensing unit (1) or with one of the dispensing units (1), and
a rotation element (31), which is rotatable concentrically around the female cone area (322) and has a rotation sleeve (311), on the outer side of which a first locking structure is formed, which is arranged in a region radially surrounding the female cone area (322) and serves for securing the attachment part (3) on the dispensing unit (1) or on one of the dispensing units (1),
wherein the rotation elements (31) of the at least two first coupling parts each have an actuating element being formed as a rotation ring (312) mounted on the rotation sleeve (311), which actuating element serves to rotate the rotation element (31) relative to the female cone area (322),
wherein in the region of the female cone areas (322) of the at least two first coupling parts in each case a shoulder (323) is formed with a first stop surface which points in a substantially opposite direction to the pushing-on direction,
and wherein the rotation elements (31) of the at least two first coupling parts each have at least one flexibly constructed behind-engaging element (313), which is arranged radially in the interior of the rotation ring (312) and extends in a direction substantially opposite to the pushing-on direction, and which has a second stop surface pointing in the pushing-on direction, and which abuts with this second stop surface against the first stop surface such that the first stop surface prevents a further movement of the rotation element (31) in the pushing-on direction.

2. The attachment part (3) according to one of the preceding claims, wherein the rotation elements (31) are each movable relative to the female cone area (322) along the pushing-on direction.

3. The attachment part (3) according to one of Claims 1 or 2, wherein an outlet opening (326) is allocated to each of the at least two first coupling parts, which is in fluid-communicating connection with the respective inlet opening (325), and wherein the attachment part (3) has a first attachment structure (327), which is suited to attach an accessory (5; 6) on the attachment part (3) such that the outlet openings (326) open in a fluid-tight manner into an opposite fluid duct of the accessory (5; 6).

4. The attachment part (3) according to one of the preceding claims, wherein the female cone areas (322) in each case form a luer cone.

5. The attachment part (3) according to one of the preceding claims, wherein the first locking structures are each constructed as an external thread.

6. The attachment part (3) according to one of the preceding claims, wherein the rotation rings (312) are each mounted over a connecting region on the rotation sleeve (311), the connecting region extending outwards perpendicularly in radial direction.

7. The attachment part (3) according to one of the preceding claims, wherein the rotation rings (312) in each case have a greater outside radius than the respective rotation sleeve (311).

8. The attachment part (3) according to one of the preceding claims, wherein the female cone areas (322) each have a conically constructed inner surface and define a longitudinal axis which extends centrally with respect to this inner surface, and wherein in each case an oblique surface is constructed at the free end of the behind-engaging elements (313), which is inclined with respect to the longitudinal axis of the respective female cone area (322) such that the oblique surface moves away from the longitudinal axis of the female cone area (322) in the direction pointing contrary to the pushing-on direction.

9. A dispensing device having an attachment part (3) according to one of the preceding claims, and a dispensing unit (1) or a plurality of dispensing units (1), which each have
a housing with at least one reservoir (16), and
at least a second coupling part with a securing sleeve (13) and with a male cone area (14), which is arranged radially at a distance within the securing sleeve (13) and has an outlet opening (15) which is in fluid-communicating connection with the reservoir (16),
wherein the female cone areas (322) of the attachment part (3) are each able to be pushed onto one of the male cone areas (14) of the dispensing unit (1) or of the dispensing units (1), so that the reservoirs (16) are each in fluid-communicating connection with one of the inlet openings (325),
and wherein the securing sleeves (13) each have a second locking structure, which is able to be locked with one of the first locking structures such that the attachment part (3) is secured on the dispensing unit (1) or on the dispensing units (1).

10. The dispensing device according to Claim 9, wherein the securing sleeves (13) are each mounted in a torque-proof manner on the respective housing.

11. The dispensing device according to one of Claims 9 or 10, comprising a dispensing unit (1) being constructed as a double- or multiple piston syringe with at least two second coupling parts arranged in parallel, and wherein the attachment part (3) has at least two first coupling parts arranged parallel to one another, wherein each of these first coupling parts is able to be coupled and locked with respectively one of the second coupling parts of the dispensing unit (1).

12. The dispensing device according to Claim 11, wherein the double- or multiple piston syringe has two or more single piston syringes (1) which are detachable from one another and are able to be used separately from one another.

13. The dispensing device according to one of Claims 11 or 12, wherein the attachment part (3) has a first attachment structure and several outlet openings (326), which are each in fluid-communicating connection with one of the inlet openings (325), wherein in addition the dispensing device has a mixing attachment (5) with a second attachment structure and with a mixing chamber (51), and wherein the mixing attachment (5) is able to be attached to the attachment part (3) by connecting the first attachment structure (327) with the second attachment structure such that the outlet openings (326) of the attachment part (3) are respectively in fluid-communicating connection with the mixing chamber (51).

## Revendications

1. Une pièce de raccordement (3) ayant au moins deux premières pièces d'accouplement disposées en parallèle l'une par rapport à l'autre, au moyen desquelles la pièce de raccordement (3) peut être raccordée à une unité de déversement (1) ou une pluralité d' unités de déversement (1), où au moins les deux premières pièces d'accouplement présentent respectivement
un domaine conique femelle (322) doté d'un orifice d'entrée traversant (325), lequel peut être coulissé le long d'une direction de coulissement sur un domaine conique mâle (14) de l'unité de déversement (1) ou une des unités de déversement (1), pour ainsi connecter la pièce de raccordement (3) avec l'unité de déversement (1) ou une des unités de déversement (1), et
un élément rotatif (31), qui peut être tourné de manière concentrique autour du domaine conique femelle (322) et qui comprend un manchon rotatif (311), à l'extérieur duquel une première structure de verrouillage est formée, laquelle est disposée dans un domaine entourant de manière radiale le domaine conique femelle (322) et qui sert à sécuriser la pièce d'accouplement (3) à l'unité de déversement (1) ou une des unités de déversement (1),
où les éléments rotatifs (31) des au moins deux premières pièces d'accouplement présentent respectivement un élément d'actionnement (312) formé en anneau rotatif (312) et apposé au manchon rotatif (311), lequel sert à tourner l'élément rotatif (31) par rapport au domaine conique femelle (322),
où dans le domaine des domaines coniques femelles (322) des au moins deux premières pièces d'accouplement un palier (323) ayant une première surface de butée est respectivement formé, laquelle est orientée en une direction essentiellement opposée à la direction de coulissement,
et où les éléments rotatifs (31) des au moins deux premières pièces d'accouplement présentent respectivement au moins un élément venant en prise par derrière (313) formé de manière flexible, lequel est disposé de manière radiale à l'intérieur de l'anneau rotatif (312) et qui s'étend en direction essentiellement opposée à la direction de coulissement, et lequel présente une deuxième surface de butée orientée en direction de coulissement et qui avec cette deuxième surface de butée bute à la première surface de butée de sorte que la première surface de butée empêche un mouvement supplémentaire de l'élément rotatif (31) en direction de coulissement.

2. Une pièce de raccordement (3) selon une des revendications précédentes, où les éléments rotatifs (31) sont respectivement mobiles le long de la direction de coulissement par rapport au domaine conique femelle (322).

3. Une pièce de raccordement (3) selon une des revendications 1 ou 2, où une ouverture de sortie est attribuée respectivement à au moins les deux premières pièces d'accouplement (326), laquelle est en communication fluidiquement communiquante avec l'ouverture de sortie (325) respective, et où la pièce de raccordement (3) présente une première structure de raccordement (327), laquelle est configurée pour raccorder une pièce accessoire (5; 6) à la pièce de raccordement (3) de sorte que les ouvertures de sortie (326) débouchent dans une conduite fluidique de la pièce accessoire (5 ; 6) opposée de manière étanche au fluides.

4. Une pièce de raccordement (3) selon une des revendications précédentes, où les domaines coniques femelles (322) forment respectivement un cône Luer.

5. Une pièce de raccordement (3) selon une des revendications précédentes, où les premières structures de verrouillage sont respectivement formées comme filetage extérieur.

6. Une pièce de raccordement (3) selon une des revendications précédentes, où les anneaux rotatifs (312) sont respectivement apposés aux manchons rotatifs (311) à travers un domaine de connexion s'étendant en direction radiale verticalement vers l'extérieur.

7. Une pièce de raccordement (3) selon une des revendications précédentes, où les anneaux rotatifs (312) présentent respectivement un rayon extérieur supérieur à celui du manchon rotatif (311) respectif.

8. Une pièce de raccordement (3) selon une des revendications précédentes, où les domaines femelles coniques (322) présentent respectivement une surface intérieure formée de manière conique et définissent un axe longitudinal, lequel s'étend de manière centrique par rapport à cette surface intérieure, et où à l'extrémité libre des éléments venant en prise par derrière (313) un plan incliné est respectivement formé, lequel est incliné de telle sorte par rapport à l'axe longitudinal du domaine conique femelle (322) respectif que le plan incliné s'éloigne de l'axe longitudinal du domaine conique femelle (322) en direction orientée de manière opposée à la direction de coulissement.

9. Un dispositif de déversement présentant une pièce de raccordement (3) selon une des revendications précédentes, ainsi qu'une unité de déversement (1) ou une pluralité d'unités de déversement (1), lesquelles présentent respectivement
un boîtier ayant au moins un réservoir (16) et
au moins une deuxième pièce d'accouplement ayant un manchon de sécurité (13) et ayant un domaine conique mâle (14), lequel est disposé de manière radialement espacée à l'intérieur du manchon du sécurité (13) et présente une ouverture de sortie (15) traversante, laquelle est en communication fluidiquement communiquante avec le réservoir (16),
où les domaines coniques femelles (322) de la pièce de raccordement (3) peuvent être coulissés respectivement sur un des domaines coniques mâles (14) de l'unité de déversement (1) respectivement des unités de déversement (1), de sorte que les réservoirs (16) sont respectivement en communication fluidiquement communiquante avec une des ouvertures de sortie (325),
et où les manchons de sécurité (13) présentent respectivement une deuxième structure de verrouillage, laquelle peut être verrouillée avec une des premières structures de verrouillage de sorte que la pièce de raccordement (3) est sécurisée à l'unité de déversement (1) respectivement à une des unités de déversement (1).

10. Un dispositif de déversement selon la revendication 9, où les manchons de sécurité (13) sont respectivement apposés de manière immobile en rotation au boîtier respectif.

11. Un dispositif de déversement selon une des revendication 9 ou 10, présentant une unité de déversement (1), laquelle est formée comme seringue à double piston ou plusieurs pistons ayant au moins deux deuxièmes pièces d'accouplement disposées en parallèle, et où la pièce de raccordement (3) présente au moins deux premières pièces d'accouplement disposées en parallèle l'une par rapport à l'autre, où chacune de ces premières pièces d'accouplement peut être verrouillée et accouplée respectivement à une des deuxièmes pièces d'accouplement de l'unité de déversement (1).

12. Un dispositif de déversement selon la revendication 11, où une seringue à double piston ou plusieurs pistons présente deux ou plusieurs seringues à piston unique pouvant être séparées et utilisées séparément l'une à l'autre.

13. Dispositif de déversement selon une des revendication 11 ou 12, où la pièce de raccordement (3) présente une première structure de raccordement et plusieurs ouvertures de sortie (326), lesquelles sont en communication fluidiquement communiquante avec respectivement une des ouvertures d'entrée (325), où en outre le dispositif de déversement présente un embout mélangeur (5) ayant une deuxième structure de raccordement et une chambre de mélange (51), et où l'embout mélangeur (5) peut être raccordé à la pièce de raccordement (3) par connexion de la première structure de raccordement (327) avec la deuxième structure de raccordement de sorte que les ouvertures de sortie (326) de la pièce de raccordement (3) sont respectivement en communication fluidiquement communiquante avec la chambre de mélange (51).
